(19) **Europäisches Patentamt / European Patent Office / Office européen des brevets**

(11) **EP 1 646 604 B1**

(12) **EUROPÄISCHE PATENTSCHRIFT**

(45) Veröffentlichungstag und Bekanntmachung des Hinweises auf die Patenterteilung:
**02.01.2008 Patentblatt 2008/01**

(51) Int Cl.:
*C07C 209/78* (2006.01)    *C07C 209/86* (2006.01)
*C07C 211/50* (2006.01)

(21) Anmeldenummer: **04740321.7**

(22) Anmeldetag: **25.06.2004**

(86) Internationale Anmeldenummer:
**PCT/EP2004/006912**

(87) Internationale Veröffentlichungsnummer:
**WO 2005/007613 (27.01.2005 Gazette 2005/04)**

(54) **VERFAHREN ZUR HERSTELLUNG VON DIAMINODIARYLMETHANEN**

METHOD FOR THE PRODUCTION OF DIAMINODIARYLMETHANES

PROCEDE DE PRODUCTION DE DIAMINODIARYLMETHANES

(84) Benannte Vertragsstaaten:
**AT BE BG CH CY CZ DE DK EE ES FI FR GB GR HU IE IT LI LU MC NL PL PT RO SE SI SK TR**

(30) Priorität: **11.07.2003 DE 10331772**

(43) Veröffentlichungstag der Anmeldung:
**19.04.2006 Patentblatt 2006/16**

(73) Patentinhaber: **BASF Aktiengesellschaft**
**67056 Ludwigshafen (DE)**

(72) Erfinder:
• **STEINBRENNER, Ulrich**
**67435 Neustadt (DE)**
• **STROEFER, Eckhard**
**68163 Mannheim (DE)**
• **SOHN, Martin**
**68229 Mannheim (DE)**
• **QUASCHNING, Veronika**
**68163 Mannheim (DE)**

• **MÖHWALD, Helmut**
**76855 Annweiler (DE)**
• **THIELE, Kai**
**01987 Schwarzheide (DE)**
• **DEBERDT, Filip**
**B-2812 Muizen (BE)**
• **JACOBS, Jan, D.**
**Baton Rouge, LA 70810 (US)**
• **PINKOS, Rolf**
**67098 Bad Dürkheim (DE)**

(56) Entgegenhaltungen:
**EP-A- 0 109 931        EP-A- 0 329 075**
**EP-A- 0 462 697        WO-A-94/23099**
**WO-A-99/40059          DE-A- 19 613 554**

• **EUGEN MÜLLER: "METHODEN DER ORGANISCHEN CHEMIE, Band I/1" 1958, GEORG THIEME VERLAG , STUTTGART (DE) , XP002306225 Seite 557 - Seite 558**

EP 1 646 604 B1

**Beschreibung**

Die vorliegende Erfindung betrifft ein Verfahren zur Herstellung von Diaminodiarylmethanen.

Diaminodiarylmethane werden zumeist hergestellt durch Kondensation der entsprechenden Amine mit Formaldehyd oder seinen Speicherverbindungen. Diese Speicherverbindungen sind beispielsweise handelsübliche wässrige Formalinlösungen, Paraformaldehyd, Trioxan oder hochkonzentrierte Formalinlösungen, wie sie in EP 1 167 343, EP 1 063 221 oder DE 100 27 778 beschrieben sind.

Zur Gewährleistung eines vollständigen Umsatzes von intermediär entstehenden Verbindungen, beispielsweise Aminobenzylanilinen, ist der Einsatz eines sauren Katalysators nötig. Das entstehende Roh - Diaminodiarylmethan besteht aus einer Mischung von Zwei- und Dreiringverbindungen und höheren Oligomeren. Die Verbindungen liegen zumeist als ortho- und para-Isomere vor.

Ein Beispiel für Diaminodiarylmethane ist 3,3'-dimethyl-4,4'-diamino-diphenylmethan, auch als Toluidinbase bezeichnet. Diese Verbindung kann als Vorprodukt für kernhydrierte Spezialamine eingesetzt werden, die vor allem als Epoxidharzhärter zur Herstellung von glasklarem Polyamid dienen. Eine weitere Einsatzmöglichkeit ist die Umsetzung der Toluidinbase mit Phosgen zu dem entsprechenden Spezialisocyanat. Ein Verfahren zur Herstellung dieses Produktes nach dem Semibatch-Verfahren ist in DE 101 16 316 beschrieben.

Ein weiteres technisch wichtiges Diaminodiarylmethan ist Diaminodiphenylmethan, auch als Methylendianilin (MDA) bezeichnet. Diese Verbindung dient vorwiegend als Vorprodukt zur Herstellung des entsprechenden Isocyanats Diphenylmethandiisocyanat (MDI), welches zumeist zur Herstellung von Polyurethanen eingesetzt wird. Die Herstellung von MDA kann kontinuierlich oder halbkontinuierlich erfolgen und ist vielfach in der Literatur beschrieben. Ein Semibatch-Verfahren zur Herstellung dieses Produktes ist in US 6,433,219 beschrieben.

Bei den genannten Verbindungen ist es wünschenswert, einen möglicht hohen Gehalt an Zweikern-Verbindungen im Reaktionsprodukt zu erhalten. Bei MDI besitzt das Zweikern-Produkt, und hier insbesondere das 4,4'-Isomere, eine große technische Bedeutung. Im Falle von Toluidinbase besteht überhaupt nur ein wesentlicher Markt für die 4,4' Zweiringverbindung.

Diese Zweiringverbindungen sind, eventuell auch nach erfolgter Kernhydrierung, einer Phosgenierung nach einem Gasphasenverfahren zugänglich, wie z.B. in EP 570 799 beschrieben. Höhere Oligomere sind der Phosgenierung in einem Gasphasenverfahren nicht zugänglich, da sie nicht unzersetzt verdampft werden können. Hersteller von Polyurethanen setzen in zunehmendem Maße bevorzugt Zweiringverbindungen ein, da diese eine geringere Viskosität aufweisen als höhere Oligomere. Ferner können sie bei ihrer Herstellung sowohl als MDI als auch als MDA durch Destillation gereinigt werden. Die Farbzahlen der aus Zweikern-MDI hergestellten Polyurethane sind generell niedriger als die Farbzahlen der aus höheren Oligomeren, auch als Roh-MDI bezeichnet, hergestellten Polyurethane. Aus den Zweikernverbindungen können durch Umsetzung mit Polyolen Prepolymere hergestellt werden, die für viele Anwendungsgebiete bei der Herstellung von Polyurethanen geeignet sind.

Somit besteht ein hoher Bedarf für Zweikernverbindungen, und bei diesen besonders für die para-Isomere. Diese sollten mit hoher Selektivität zu eben diesen Verbindungen in der Stufe der Aminkondensation hergestellt werden. Durch einen erhöhten Gehalt an saurem Katalysator im Reaktionsgemisch in der Kondensationsstufe kann die Reaktion in Richtung eines erhöhten Anteils an Zweikern-Produkt und hierbei besonders der 4,4' Zweiringverbindung verschoben werden. Ein weiterer Vorteil eines erhöhten Einsatzes von Säure ist die Erhöhung der Reaktionsgeschwindigkeit. Üblicherweise wird die Säure nach der Kondensation mit Basen neutralisiert und die entstehenden Salze abgetrennt. Dies führt zu einem hohen Verbrauch an Säure und Neutralisationsmitteln sowie zu einer unerwünschten Salzlast.

DE-A-196 13 554 beschreibt ein Verfahren zur Herstellung von MDA durch Umsetzung von Anilin und Formaldehyd unter Verwendung von Mineralsäuren als Katalysatoren, bei dem die Säure nach der Umsetzung im wesentlichen durch Extraktion entfernt wird. Eventuell im Reaktionsgemisch verbleibende Säurespuren können durch einen basischen Ionenaustauscher entfernt werden.

Eugen Müller, "Methoden der organischen Chemie, Band I/1", Georg-Thieme-Verlag, 1958, Seiten 557-558 offenbaren allgemein die Entfernung von Säuren aus organischen Gemischen mittels Ionenaustauschern.

EP 109 931 beschreibt die Herstellung und Verwendung von MDA, wobei keine näheren Angaben zur Entfernung der Säure aus dem Reaktionsgemisch gemacht wurden.

Die Säure kann nach der Kondensation auch durch Extraktion mit Wasser aus dem Kondensat entfernt werden. Dabei sinkt jedoch die Raum-Zeit-Ausbeute, und die Kreislaufströme steigen an.

In WO 01/58847 wird ein Verfahren zur Herstellung von MDA mit einem hohen Gehalt an Zweikern-Anteilen beschrieben, bei dem ein getrocknetes Kondensat von Anilin und der Darreichungsform des Formaldehyds bei einem molaren Verhältnis von 1,7 bis 100 in Gegenwart fester, anorganischer, saurer Katalysatoren umgesetzt wird. Dadurch sollen die Nachteile bei der Verfahrensführung mit Verwendung von Mineralsäuren, insbesondere der erhöhte Verbrauch an Säure, die anschließend aufwendig aus dem Reaktionsgemisch entfernt werden muss, vermieden werden.

Das dort beschriebene Verfahren weist jedoch auch Nachteile auf. Dies sind insbesondere die ungenügende Standzeit des Katalysators durch Desaktivierung infolge Belagsbildung mit Oligomeren, die Titration der Säuregruppen des Katalysators mit im Anilin enthaltenen oder während der Reaktion gebildeten sekundären Aminen, wie N-Methylaminen, sowie die hohen Kosten für den Katalysator und für durch Wechsel und Regenerierung des Katalysators bedingte Stillstände, die durch eine entsprechende Standzeit des Katalysators ausgeglichen werden müssen.

Es wäre demzufolge wünschenswert, ein Verfahren zur Herstellung von Diaminodiarylmethanen mit einem hohen Gehalt an Zweikern-Anteilen zu entwickeln, bei dem die Säure als homogener Katalysator eingesetzt wird, ohne dass eine aufwendige Entfernung der Säure mit den damit verbundenen Nachteilen wie dem hohen Säureverbrauch und der erhöhten Salzfracht im Abwasser notwendig ist.

Diese Aufgabe konnte überraschenderweise dadurch gelöst werden, dass die Säure nach der Umsetzung ganz oder teilweise an einem basischen Ionenaustauscher adsorbiert wird.

Gegenstand der Erfindung ist somit ein Verfahren zur Herstellung von Diaminodiarylmethanen, umfassend die Schritte

   a) Umsetzung eines aromatischen Amins mit einem Methylengruppen liefernden Agens in Gegenwart von homogenen sauren Katalysatoren,

   b) Entfernung des homogenen sauren Katalysators aus dem Umsetzungsprodukt, und gegebenenfalls

   c) Aufarbeitung und Reinigung des Umsetzungsprodukts,

dadurch gekennzeichnet, dass die Entfernung des homogenen sauren Katalysators aus dem Reaktionsgemisch durch Adsorption an einem festen Adsorptionsmittel erfolgt, und die Regenierung des Adsorptionsmittels mit dem Amin erfolgt, das als Einsatzprodukt des Verfahrens verwendet wird.

Gegebenenfalls ist es möglich, das Reaktionsgemisch nach der Adsorption des homogenen sauren Katalysators noch einer Nachneutralisation mit üblichen basischen Neutralisationsmitteln, wie Aminen oder Alkalihydroxiden, zu unterziehen, um letzte Spuren der Säure zu entfernen. Die hierbei anfallende Salzlast ist jedoch wesentlich geringer als bei einer Neutralisation der gesamten Menge des homogenen sauren Katalysators mit basischen Neutralisationsmitteln. Für bestimmte Einsatzgebiete der Diaminodiarylmethane, beispielsweise der Herstellung von Polyisocyanaten, kann auch ein Restgehalt an Säure im Produkt verbleiben.

Das Adsorptionsmittel ist vorzugsweise ein auf Basis von höheren Oligomeren des Diphenylmethandiamins oder auf Basis eines aktive basische Zentren aufweisenden anorganischen oder organischen Trägermaterials hergestellter basischer Ionentauscher.

Die Basenstärke dieser Ionenaustauscher weicht vorzugsweise +/- 1,0 $pK_B$-Einheiten, insbesondere +/- 0,5 $pK_B$-Einheiten von der des aromatischen Amins in wässriger Lösung ab. Die Bestimmung der Basenstärke des Amins und des Ionenaustauschers kann mittels Säure-Base-Titration erfolgen.

In einer bevorzugten Ausführungsform bestehen die aktiven basischen Zentren des Adsorptionsmittels zu > 80 %, bevorzugt > 90 %, insbesondere > 95 % aus aromatischen oder aliphatischen Aminen, insbesondere Anilin- und Toluidineinheiten.

Der als Adsorptionsmittel eingesetzte Ionentauscher kann entweder aus auf einem Träger aufgebrachten aktiven Zentren oder aus einem Polymer oder Copolymer, enthaltend diese aktiven Zentren, bestehen. Als Träger eignen sich Polymere, übliche anorganische Träger, Aktivkohlen oder Metalle. Die Bindung der aktiven Komponenten auf diesen Trägern kann über van-der-Waals, bevorzugt über ionische und besonders bevorzugt über kovalente Bindungen, beispielsweise durch Verwendung von C-C oder O-Si-C-Brücken, erfolgen.

In einer Ausführungsform des erfindungsgemäßen Verfahrens werden als Adsorptionsmittel höhere Oligomere von Diphenylmethandiaminen eingesetzt, welche die notwendigen aktiven Zentren enthalten. Diese Oligomere können beispielsweise durch Kondensation von aromatischen Aminen mit einem Methylengruppen liefernden Agens, insbesondere in Gegenwart von homogenen sauren Katalysatoren, unter solchen Bedingungen hergestellt werden, bei denen der Aufbau von hochmolekularen Reaktionsprodukten begünstigt ist. Derartige Produkte können auch hergestellt werden, indem Kondensationsprodukte von aromatischen Aminen mit einem Methylengruppen liefernden Agens, die ein geringeres Molekulargewicht aufweisen, insbesondere solche mit 2 bis 5 aromatischen Kernen im Molekül, in geeigneter Weise miteinander vernetzt werden. Wesentlich ist, dass die Oligomere unter den Bedingungen der Adsorption fest sind. Ganz besonders bevorzugt ist die Verwendung von höheren Kondensationsprodukten aus Anilin oder Toluidin und Carbonylverbindungen, bevorzugt Ketonen und/oder Aldehyden, insbesondere Formaldehyd.

In weiterer einer Ausführungsform des erfindungsgemäßen Verfahrens werden als Adsorptionsmittel Ionentauscher einem Träger mit darauf befindlichen aktiven Zentren eingesetzt. Die aktiven Zentren können Bestandteil des Trägers oder nachträglich auf den Träger aufgebracht sein. Die Träger müssen in der letztgenannten Ausführungsform für das Aufbringen der aktiven Zentren geeignet sein.

Als polymere Träger eignen sich beispielsweise Polyethylen, Polypropylen, Polystyrol, Polyamide, Polyester, Polyether, Polysulfone, Polyethersulfone, Polyketone, Polyurethane, Polytetrafluorethylen, Polyvinylidenfluorid, Polyanilin, Polypyrrol und Polythiophen.

Geeignete polymere Träger sind auch Copolymere, erhältlich durch Polymerisation mindestens eines Kondensationsproduktes aus mindestens einer Verbindung, die in der Lage ist, mit einer Carbonsäure oder einem Derivat einer Carbonsäure zu reagieren, mindestens einem Mol pro Mol dieser Verbindung einer Carbonsäure, die mindestens eine radikalisch polymerisierbare funktionelle Gruppe aufweist, oder eines Derivats davon, und gegebenenfalls einer weiteren Verbindung mit einem mittleren Molekulargewicht (Zahlenmittel) von mindestens 5000 mit Polyethersegmenten in der Hauptkette, wie sie beispielsweise in WO 99/57161, Seiten 29 bis 30, beschrieben sind.

Eine weitere Gruppe von polymeren Trägern sind vernetzbare Polymere. Diese werden erhalten durch radikalische Polymerisation, Polyaddition oder Polykondensation von Monomerbausteinen, die neben den Gruppen, über die der Polymeraufbau erfolgt, noch eine oder mehrere weitere reaktive Gruppen aufweisen, so dass die vernetzbaren Polymere schon bei der Polymerherstellung gebildet werden. Typische Beispiele hierfür sind Polyester, die unter Verwendung von ungesättigten Carbonsäuren, beispielsweise (Meth)acrylsäure, hergestellt werden. Derartige Polymere werden beispielsweise in WO 99/57161, Seiten 22 bis 28, beschrieben.

Als anorganische Träger werden beispielsweise Oxide, wie Siliziumdioxid, Aluminiumoxid, Magnesiumoxid oder Titandioxid, Mischoxide, beispielsweise der Elemente Silizium, Calcium, Aluminium, Magnesium, Titan, Silicate wie Leiter-, Ketten-, Schicht- und Gerüstsilicate, wie Talk, Porphyrit, Muskovit, Zeolithe, Feldspäte, Wollastonit, Glimmer, Carbonate und/oder Phosphate verwendet

Der erfindungsgemäß verwendete Ionentauscher wird bevorzugt in Form von Granulat oder Extrudat eingesetzt. Das Verhältnis aus Oberfläche und Volumen der Formkörper beträgt dabei > 0,2 mm$^{-1}$, bevorzugt > 0,5 mm$^{-1}$, besonders bevorzugt > 1 mm$^{-1}$. Das Partikelvolumen beträgt < 300 mm$^3$, bevorzugt < 100 mm$^3$, besonders bevorzugt < 50 mm$^3$. Der Feinanteil, das sind Partikel mit < 0,1 mm$^3$, beträgt < 10 Gew.-%, bevorzugt < 5 Gew.-%.

Die Formkörper des Ionentauschers werden vorzugsweise in basischem Zustand in ein Festbett eingebracht. Der Ionentauscher kann in einem Zwei- oder Dreiphasencyclus aus Adsorption, optional Spülung und Regenerierung betrieben werden.

Die jeweiligen Fließgeschwindigkeiten liegen dabei zumeist unter 30 Bettvolumen, bevorzugt < 10 Bettvolumen, besonders bevorzugt < 5 Bettvolumen pro Stunde.

Der Ionentauscher besitzt eine Porosität bzw. in der Reaktionsmischung ein Quellverhalten, so dass sich im Betrieb für im Formkörper diffundierendes, reines Anilin bei 20°C ein scheinbarer Diffusionskoeffizient > $10^{-8}$ cm$^2$/s, bevorzugt > $5*10^{-7}$ cm$^2$/s ergibt. Der scheinbare Diffusionskoeffizient ist dabei definiert als

$$D = \frac{I}{d\vec{A} \cdot \vec{\nabla}c}$$ mit dem messbaren Strom I [mol/s] durch ein kleines Flächenelement dA und der Konzentration an Amin c [mol/l] bezogen auf die freie flüssige Phase, d.h. ohne Berücksichtigung des Volumens, welches durch den Ionentauscher eingenommen wird. D kann leicht durch Methoden wie Pulsed Field-Gradient NMR oder Austauschexperimente bestimmt werden.

Wie beschrieben, erfolgt die Aufarbeitung des Rektionsgemisches aus der Umsetzung der aromatischen Amine mit dem Methylengruppen liefernden Agens durch die Entfernung des sauren Katalysators mit einem Adsorptionsmittel.

Dabei wird im ersten Schritt das zurückzugewinnende Säureanion enthaltende Reaktionsgemisch über das Adsorptionsmittel geleitet. Die Adsorbierphase wird beendet, sobald die Konzentration des Säureanions in der austretenden Lösung > 50 %, bevorzugt > 10 %, besonders bevorzugt > 1 % der eintretenden Lösung ist. Der Restgehalt an Säure, der im Reaktionsgemisch verbleiben kann, richtet sich nach dem beabsichtigten Einsatzzweck des Diaminodiarylmethans. Entsprechend kann der Apparat, insbesondere das Festbett, in dem sich das Adsorptionsmittel befindet, ausgelegt werden. Es ist auch möglich, mehrere derartige Apparate hintereinander anzuordnen, oder mehrere derartige Apparate parallel zu schalten, um den Produktstrom bei Bedarf von einem auf den anderen Apparat umzuschalten.

Wenn die Aufnahmekapazität des Adsorptionsmittels erreicht ist, erfolgt seine Regenerierung. Vor der Regenerierung kann eine Spülphase durchgeführt werden. Dabei wird das im Ionentauscherbett verbliebene Produkt im Gegenstrom mit einer Base, insbesondere einem Amin, bevorzugt dem als Edukt eingesetzten Amin, in den mit Säure beladenen Produktstrom ausgespült. Dadurch werden auch Diaminodiarylmethan und andere Bestandteile des Produktstroms, die sich an der Oberfläche des Adsorptionsmittels abgesetzt haben und somit dessen Wirksamkeit beeinträchtigen, entfernt.

Die Regenerierung erfolgt insbesondere durch Behandlung mit dem Amin vorzugsweise im Gegenstrom. Zur Regeneration wird das Einsatzprodukt des erfindungsgemäßen Verfahrens verwendet, also bei der Herstellung von MDA Anilin und bei der Herstellung von Toluidinbase Toluidin. Das mit der Säure beladene Amin kann, gegebenenfalls im Gemisch mit dem bei der Spülung anfallenden Produktstrom, von der Regenerierung direkt dem erfindungsgemäßen Verfahren wieder als Einsatzprodukt zugeführt werden, wobei gegebenenfalls durch Zusatz weiterer Säure oder Abtrennung, bevorzugt destillativer Abtrennung von Amin das für das Verfahren notwendige Verhältnis von Säure zu Amin eingestellt werden kann. Durch diese Ausgestaltung des Verfahrens kann der überwiegende Teil der Säure im Kreislauf gefahren werden, was zu einer Einsparung an Säure und Neutralisationsmittel sowie zu einer Verringerung der Menge an Abfallprodukten führt.

Die Regenerierung ist beendet, wenn die Konzentration des Säureanions in der austretenden Lösung < 20 %, bevorzugt < 10 %, besonders bevorzugt < 1 % der zu Beginn der Regenerationsprozesses in der austretenden Lösung enthaltenen Konzentration der Säureanionen ist.

Regenerierlösungen und gegebenenfalls Spüllösungen werden durch Destillation und/oder Aufstocken mit Amin und/oder Säure wieder zu einer für die vorgeschaltete Formaldehydkondensation geeigneten Zusammensetzung konditioniert.

Die Herstellung der Diaminodiarylmethane erfolgt, wie oben beschrieben, durch Umsetzung eines aromatischen Amins mit einem Methylengruppen liefernden Agens in Gegenwart von homogenen sauren Katalysatoren. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

Als Methylengruppen lieferndes Agens wird vorzugsweise Formaldehyd oder eine Formaldehyd abspaltende Verbindung eingesetzt. Insbesondere wird der Formaldehyd als wässrige Formalinlösung, alkoholische Formalinlösung, Halbacetal, Methylenimin eines primären Amins oder N,N'-Methylendiamin eines primären oder sekundären Amins sowie Paraformaldehyd eingesetzt. Bevorzugt sind wässrige Formalinlösung und Methylenimin oder Di-, Oligo- oder Polymere N,N'-Methylendiamine der Einsatzprodukte des erfindungsgemäßen Verfahrens, also bei der Herstellung von MDA Anilin und bei der Herstellung von Toluidinbase Toluidin.

Als homogene saure Katalysatoren werden insbesondere Mineralsäuren, und hier bevorzugt Salzsäure, eingesetzt.

Das erfindungsgemäße Verfahren kann kontinuierlich, halbkontinuierlich oder batchweise, vorzugsweise kontinuierlich oder halbkontinuierlich, durchgeführt werden.

Bei der kontinuierlichen Fahrweise werden die Reaktanden in dem gewünschten Verhältnis zueinander in einen Reaktor eindosiert und diesem Reaktor eine dem Zustrom gleiche Menge an Reaktionsprodukt entnommen. Als Reaktoren kommen beispielsweise Rohrreaktoren zum Einsatz. Bei der kontinuierlichen oder halbkontinuierlichen Fahrweise werden die Reaktanden in einen vorzugsweise mit einem Rührer und/oder einem Umpumpkreis versehenen Batchreaktor dosiert, aus dem das ausreagierte Reaktionsprodukt entnommen und der Aufarbeitung zugeführt wird.

Das erfindungsgemäße Verfahren wird vorzugsweise bei einem molaren Verhältnis von Anilin zu Formaldehyd größer 2 durchgeführt. Das molare Verhältnis von Säure zu Anilin ist vorzugsweise größer 0,05. Bei diesen Verhältnissen kommt es zu einer verstärkten Bildung der jeweiligen Zweikernprodukte in der Reaktionsmischung.

Die Reaktion wird vorzugsweise bei einer Temperatur im Bereich zwischen 0 und 200°C, vorzugsweise zwischen 20 und 150°C und insbesondere zwischen 40 und 120°C durchgeführt. Es hat sich gezeigt, dass mit der Erhöhung der Temperatur der Anteil der 2,2'- und 2,4'-Isomeren im Reaktionsprodukt ansteigt.

Der Druck bei der Umsetzung beträgt 0,1 bis 50, bevorzugt 1 bis 10 bar absolut.

Bei der batchweisen und halbkontinuierlichen Durchführung der Reaktion kann nach der vollständigen Dosierung der Einsatzstoffe das Reaktionsgemisch einer sogenannten Alterung unterzogen werden. Dazu wird das Reaktionsgemisch im Reaktor belassen oder in einen anderen, vorzugsweise gerührten Reaktor überführt. Dabei liegt die Temperatur des Reaktionsgemisches vorzugsweise über 75°C, insbesondere in einem Bereich zwischen 110 und 150°C.

An die Herstellung des Kondensationsprodukts schließt sich die erfindungsgemäße, oben näher beschriebene Aufarbeitung an.

Verbliebene Spuren an Säure im Reaktionsprodukt können mit Lauge neutralisiert werden. Die weitere Aufarbeitung der Produktmischungen erfolgt wie bei herkömmlichen Verfahren zur Herstellung derartiger Kondensationsprodukte, beispielsweise durch Phasentrennung, Destillation, und/oder chromatographische Trennmethoden.

Die so hergestellten und aufgearbeiteten Diaminodiarylmethane können nach den üblichen und bekannten Verfahren weiterverarbeitet werden.

Das MDA kann beispielsweise durch Umsetzung mit Alkylenoxiden zu Polyetheralkholen weiterverarbeitet werden.

Hauptsächlich wird das MDA mit Phosgen zu MDI umgesetzt. Derartige Verfahren sind allgemein bekannt und vielfach beschrieben, beispielsweise im Kunststoffhandbuch, Band 7, Polyurethane, Carl Hanser Verlag München Wien, 3. Auflage, 1993, S. 76-86, sowie in einer großen Zahl von Patentanmeldungen, beispielsweise WO 99/40059 oder WO 99/54289.

Dazu wird üblicherweise das MDA und gegebenenfalls das Phosgen in einem inerten Lösungsmittel gelöst und zur Reaktion gebracht.

Das genannte Verfahren kann in üblichen Reaktoren, beispielsweise Rührkesseln, Rührkesselkaskaden, Kolonnen und/oder Rohrreaktoren bei bekannten Temperaturen von z.B. 50 bis 150°C, bevorzugt 70 bis 120°C, besonders bevorzugt 70 bis 100°C und einem Druck von 0,5 bis 10 bar, bevorzugt 0,8 bis 5 bar, besonders bevorzugt 0,8 bis 1,5 bar in einer oder mehreren Stufen durchgeführt werden.

Beispielsweise kann die Phosgenierung durchgeführt werden durch eine zweistufige Umsetzung in Gegenwart mindestens eines inerten organischen Lösungsmittels, wobei die erste Stufe der Phosgenierung in einem statischen Mischer und die zweite Stufe der Phosgenierung in einem Verweilzeitapparat durchgeführt werden.

Das durch die Phosgenierung hergestellte Roh-MDI kann durch übliche Verfahren, beispielsweise Destillation, gereinigt werden. Bevorzugt kann in einem ersten Reinigungsvorgang Phosgen und gegebenenfalls Lösungsmittel, bevorzugt weitgehend, besonders bevorzugt vollständig aus dem Reaktionsgemisch der Phosgenierung, d.h. dem Roh-MDI entfernt werden.

Bevorzugt kann anschließend gewünschtes monomeres MDI, beispielsweise 2,2'-, 2,4'- und/oder 4,4'-MDI und/oder Gemische enthaltend mindestens zwei diese Isomere, durch ein geeignetes Verfahren, bevorzugt durch Destillation, beispielsweise bei Drücken von 2 bis 50 mbar, bevorzugt 2 bis 20 mbar, und Temperaturen von 150 bis 250°C, bevorzugt 180 bis 230°C, und/oder bevorzugt durch Kristallisation, beispielsweise fraktionierte Kristallisation, abgetrennt werden.

In einer besonderen Ausführungsform des Verfahrens zur Herstellung von MDI kann aus dem Roh-MDA das Zweikern-Produkt abgetrennt und mittels Gasphasenphosgenierung, wie beispielsweise EP 570 799 beschrieben, zu Zweikern-MDI umgesetzt werden.

Das so hergestellte MDI kann insbesondere mit Verbindungen mit mindestens zwei aktiven Wasserstoffatomen zu Polyurethanen umgesetzt werden.

Durch das erfindungsgemäße Verfahren ist es möglich, bei der Herstellung von Diaminodiarylmethanen die benötigte Menge an Säure deutlich zu reduzieren. Dadurch können die Herstellungskosten gesenkt und die als Abfallprodukt entstehende Salzmenge reduziert werden.

**Patentansprüche**

1. Verfahren zur Herstellung von Diaminodiarylmethanen, umfassend die Schritte

    a) Umsetzung eines aromatischen Amins mit einem Methylengruppen liefernden Agens in Gegenwart von homogenen sauren Katalysatoren,
    b) Entfernung des homogenen sauren Katalysators aus dem Umsetzungsprodukt,
    c) Aufarbeitung und Reinigen des Umsetzungsprodukts.

    **dadurch gekennzeichnet, dass** der homogene saure Katalysator durch Adsorption an einem festen Adsorptionsmittel aus dem Reaktionsgemisch entfernt wird und die Regenerierung des Adsorptionsmittels mit dem Amin erfolgt, das als Einsatzprodukt des Verfahrens verwendet wird.

2. Verfahren nach Anspruch 1, **dadurch gekennzeichnet, dass** das Adsorptionsmittel ein auf Basis von höheren Oligomeren mit 2 bis 5 aromatischen Kernen im Molekül des Diphenylmethandiamins oder auf Basis eines funktionalisierten-Trägermaterials hergestellter basischer Ionentauscher ist.

3. Verfahren nach Anspruch 1, **dadurch gekennzeichnet, dass** die Basenstärke des Adsorptionsmlttels +/-1,0 $pK_B$-Einheiten von der des aromatischen Amins in wässriger Lösung abweicht.

4. Verfahren nach Anspruch 1, **dadurch gekennzeichnet, dass** die Basenstärke des Adsorptionsmittels +/- 0,5 $pK_B$-Einheiten von der des aromatischen Amins in wässriger Lösung abweicht.

5. Verfahren nach Anspruch 1, **dadurch gekennzeichnet, dass** der saure Homogenkataysator mit dem aromatischen Amin desorbiert und in die Umsetzung zurückgeführt wird.

6. Verfahren nach Anspruch 1, **dadurch gekennzeichnet, dass** die Umsetzung in Schritt a) halbkontinuierlich durchgeführt wird.

7. Verfahren nach Anspruch 1, **dadurch gekennzeichnet, dass** das aromatische Amin ausgewählt ist aus der Gruppe, enthaltend Anilin und Alkylaniline mit 1 bis 3 Kohlenstoffatomen in der Alkylkette.

8. Verfahren nach Anspruch 1, **dadurch gekennzeichnet, dass** das aromatische Amin ausgewählt ist aus der Gruppe, enthaltend Anilin und o-Toluidin.

9. Verfahren nach Anspruch 1, **dadurch gekennzeichnet, dass** das Methylengruppen liefernden Agens Formaldehyd ist.

10. Verfahren nach Anspruch 1, **dadurch gekennzeichnet, dass** das Formaldehyd als wässrige Formalinlösung oder Paraformaldehyd eingesetzt wird.

11. Verfahren nach Anspruch 1, **dadurch gekennzeichnet, dass** das molare Verhältnis von Anilin zu Formaldehyd größer 2 ist

12. Verfahren nach Anspruch 1, **dadurch gekennzeichnet, dass** das molare Verhältnis von Säure zu Anilin größer 0,05 ist.

13. Verfahren nach Anspruch 1, **dadurch gekennzeichnet, dass** als homogene saure Katalysatoren Mineralsäuren eingesetzt werden.

**Claims**

1. A process for preparing diaminodiarylmethanes comprising the steps

    a) reacting an aromatic amine with a methylene-donating agent in the presence of homogeneous

# EP 1 646 604 B1

acid catalysts,

b) removing the homogeneous acid catalyst from the reaction product,

c) working up and purifying the reaction product,

which comprises removing the homogeneous acid catalyst from the reaction mixture by adsorption to a solid adsorbent and the absorbent is regenerated with the amine which is used as the feed product of the process.

2. The process according to claim 1, wherein the adsorbent is a basic ion exchanger prepared on the basis of higher oligomers having 2 to 5 aromatic nuclei in the molecule of diphenylmethanediamine or on the basis of functionalized support material.

3. The process according to claim 1, wherein the base strength of the adsorbent differs by +/- 1.0 $pK_B$ units from that of the aromatic amine in aqueous solution.

4. The process according to claim 1, wherein the base strength of the adsorbent differs by +/- 0.5 $pK_B$ units from that of the aromatic amine in aqueous solution.

5. The process according to claim 1, wherein the acid homogeneous catalyst is desorbed by the aromatic amine and recirculated to the reaction.

6. The process according to claim 1, wherein the reaction in step a) is carried out semicontinuously.

7. The process according to claim 1, wherein the aromatic amine is selected from the group comprising aniline and alkylanilines having from 1 to 3 carbons in the alkyl chain.

8. The process according to claim 1, wherein the aromatic amine is selected from the group comprising aniline and o-toluidine.

9. The process according to claim 1, wherein the methylene-donating agent is formaldehyde.

10. The process according to claim 1, wherein the formaldehyde is used as aqueous formalin solution or paraformaldehyde.

11. The process according to claim 1, wherein the molar ratio of aniline to formaldedhyde is greater than 2.

12. The process according to claim 1, wherein the molar ratio of acid to aniline is greater than 0.05.

13. The process according to claim 1, wherein mineral acids are used as homogeneous acid catalysts.

## Revendications

1. Procédé de production de diaminodiarylméthanes, comprenant les étapes suivantes :

(a) mise en réfaction d'une amine aromatique avec un agent fourbissant des groupes méthylène en présence de catalyseurs acides homogène,

(b) élimination du catalyseur acide homogène du produit réactionnel;

(c) traitement et lavage du produit réactionnel,

**caractérisé en ce que** le catalyseur acide homogène est éliminé du mélange réactionnel par adsorption sur un agent d'adsorption solide et la dégénération de l'agent d'adsorption est effectuée avec l'amine qui est utilisée comme produit mils en oeuvre dans le procédé.

2. Procédé selon la revendication 1, **caractérisé en ce que** l'agent d'adsorption est un échangeur d'ions basique préparé à partir d'oligomères supérieurs avec 2 à 5 noyaux aromatiques dans la molécule de la diphénylméthanediamine ou à partir d'une matière de support fonctionnalisée.

3. Procédé selon la revendication 1, **caractérise en ce que** la basicité de l'agent d'adsorption s'écarte de +/- 1,0 unité $pK_B$ de celle de l'amine aromatique dans la solution aqueuse.

4. Procédé selon la revendication 1, **caractérisé en ce que** la basicité de l'agent d'adsorption s'écarte de +/- 0,5 unité $pK_B$ de celle de l'aminé aromatique dans la solution aqueuse.

5. Procédé selon la revendication 1, **caractérisé en ce que** le catalyseur homogène acide est désorbé avec l'amine aromatique et est réinjecté dans la réaction.

6. Procédé selon la revendication 1, **caractérisé en ce que** la réaction dans l'étape a) est effectuée de manière semi-continue.

7. Procédé selon la revendication 1, **caractérisé en ce que** l'amine aromatique est sélectionnée dans le groupe contenant l'aniline et les alkylanilines avec 1 à 3 atomes de carbone dans la chaîne alkyle.

8. Procédé selon la revendication 1, **caractérisé en ce que** l'aminé aromatique est sélectionnée dans le groupe contenant l'aniline et la o-toluidine.

9. Procédé selon la revendication 1, **caractérisé en ce que** l'agent fournissant des groupes méthylène est le formaldéhyde.

10. Procédé selon la revendication 1, **caractérisé en ce que** le formaldéhyde est utilisé comme solution aqueuse de formaline ou paraformaldéhyde.

11. Procédé selon la revendication 1, **caractérisé en ce que** le rapport molaire de l'aniline au formaldéhyde est supérieur à 2.

12. Procédé selon la revendication 1, **caractérisé en ce que** le rapport molaire de l'acide à l'aniline est supérieur à 0,05.

13. Procédé selon la revendication 1, **caractérisé en ce que** les catalyseurs acides homogènes utilisés sont des acides minéraux.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1167343 A
- EP 1063221 A
- DE 10027778
- DE 10116316
- US 6433219 B
- EP 570799 A

- DE 19613554 A
- EP 109931 A
- WO 0158847 A
- WO 9957161 A
- WO 9940059 A
- WO 9954289 A

**In der Beschreibung aufgeführte Nicht-Patentliteratur**

- **EUGEN MÜLLER.** Methoden der organischen Chemie. Georg-Thieme-Verlag, 1958, vol. I/1, 557-558

- Polyurethane. Kunststoffhandbuch. Carl Hanser Verlag, 1993, vol. 7, 76-86
- Polyurethane. Kunststoffhandbuch. Carl Hanser Verlag, 1993, vol. 7, 76-86